# EUROPEAN PATENT SPECIFICATION

(11) **EP 2 335 703 B1**
(45) Date of publication and mention of the grant of the patent: **24.09.2014**
(21) Application number: 11159742.3
(22) Date of filing: 12.06.2007
(51) Int. Cl.: A61K 31/519, A61K 31/196, A61K 47/38, A61P 1/00

(54) **Compositions and methods for preventing or reducing postoperative ileus and gastric stasis in mammals**
Zusammensetzungen und Verfahren zur Vorbeugung oder Linderung von postoperativem Darmverschluss und Magenstasis bei Säugetieren
Compositions et procédés de prévention ou de réduction d'un ileus et d'une stase gastrique postoperatoires chez les mammifères

(30) Priority: 13.06.2006 US 813250 P
(43) Date of publication of application: 22.06.2011
(62) Divisional of application: 07812111.8
(73) Proprietor: Ethicon, Incorporated, Somerville, NJ 08876 (US)
(72) Inventor: Herzberg, Uri, Bridgewater, NJ 08807 (US); Wadsworth, Scott, New Hope, PA 18938 (US); Cooper, Kevin, Flemington, NJ 08822 (US)
(74) Representative: Kirsch, Susan Edith

(56) References cited:
- WO-A2-2007/076415
- US-A1- 2004 109 894
- LUCKEY ANDREW ET AL: "Mechanisms and treatment of postoperative ileus.", ARCHIVES OF SURGERY (CHICAGO, ILL. : 1960) FEB 2003 LNKD- PUBMED:12578422, vol. 138, no. 2, February 2003 (2003-02), pages 206-214, XP002635337, ISSN: 0004-0010
- PAIRET M ET AL: "ON THE RELEVANCE OF NON-STEROIDAL ANTI-INFLAMMATORY DRUGS IN THE PREVENTION OF PARALYTIC ILEUS IN RODENTS", JOURNAL OF PHARMACY AND PHARMACOLOGY, ROYAL PHARMACEUTICAL SOCIETY OF GREAT BRITAIN, GB, vol. 41, no. 11, 1 January 1989 (1989-01-01), pages 757-761, XP008102526, ISSN: 0022-3573

## Description

### Field

The present invention is directed to compositions for the prevention or reduction of postoperative ileus and gastric stasis in mammals.

### Background

Postoperative ileus (also referred to herein as "POI") and gastric stasis are problems that are common to most surgeries involving the abdomen. In addition, ileus and stasis are the main impediments for releasing a patient from the hospital.

Ileus is a major complication following abdominal surgery, and especially an abdominal surgery that involves manipulating the intestines and other abdominal organs. Specifically, paralytic ileus is the functional inhibition of peristaltic motility of the intestines. It prevents the absorption of drugs and nutrients, increases patient discomfort and pain, prolongs patient hospitalization and increases postoperative health care costs.

Care of the patient after surgery frequently does little to address the ileus condition and, in fact, often adds complications. Since opiates decrease intestinal motility, analgesic drugs such as morphine and codeine administered after surgery only exacerbate the severity and increase the incidence of postoperative ileus.

The main approaches for treating postoperative ileus and gastric stasis involve the use of systemic drugs. Currently there is only one drug, which has tentative approval for treating the condition. Specifically, a peripherally active opiate antagonist, Entereg (By Adolor and GlaxoSmithKline), has been submitted to the FDA for treating POI. Blocking peripheral opiate receptors is indeed a viable approach in preventing postoperative ileus. (See, for example, US 2002/0188005) Yet, this approach is only valuable in blocking the gastrointestinal effects of opiates which are commonly administered as analgesics following surgery. Such drugs are not likely to have an effect on the intestinal and gastric stasis that is opiates independent. Other approaches involve other mediators such as blocking the adenosine A1 receptor, blocking the Cox2 enzyme, and using the anti-inflammatory cytokine IL-11, etc. Blocking the PAR-2 enzyme has also been suggested as an approach (See, for example, WO 9843477.)

Common to all these approaches is the systemic delivery of an agent that enhances gastric and intestinal motility, or prevents the stasis and ileus of the stomach and intestines. However, systemic drug delivery, while simple, carries with it the side effect profile of the drug. For example, when systemically delivering anti-inflammatory drugs, such as NSAIDs, tissue healing may be affected. Cytokine injections such as IL-11 will affect the immune response of the patient, and systemic administration of A1 antagonists will have effects on the nervous system. Furthermore, for systemic drug delivery, non-target organs will also be affected.

While the pathology mediating the ileus and stasis condition is not clear, animal studies point to the activation of the cyclooxygenase enzymes (Cox1 and Cox2) as being at least partially responsible for this condition. While many Cox1 and Cox2 inhibitors are available, the systemic administration of Cox1 and Cox2 inhibitors to the post operative patient is, as with other systemically delivered agents, undesirable. This is due to their gastrointestinal side effect profile, their inhibitory effects on wound healing, their platelet inhibition and, in the case of selective COX2 inhibitors such as Vioxx and Celebrex, their cardivascular side effects.

There is also evidence suggesting that mast cell degranulation and histamine release play a role in the induction and maintenance of POI (Demol et al. 1989, de Jonge et al. 2004). Following mast cell activation, histamine release initiates an inflammatory cascade whereby Cox1 and Cox2 enzymes are activated, nitric oxide synthase is activated and receptors for these mediators convey the information to neural centers. These neural centers reduce gastric emptying and block the coordinated motility of the intestines. Yet, the application of antihistamines, or mast cell degranulation inhibitors to the manipulated intestines in levels that are pharmacologically relevant to the problem without reaching systemic levels that will cause unwanted side effects remains a challenge. Given the foregoing considerations, there is a continuing need to identify treatment or prevention methods and compositions which are suitable and effective for preventing, treating and ameliorating postoperative ileus and gastric statis. Such methods and compositions are ideally those which directly address the pathology of the condition but do not involve the systemic administration of drugs which can cause undesirable side effects.

WO-A-2007076415 forms part of the state of the art by virtue Article 54(3) EPC. It describes compositions containing oxidised regenerated cellulose and non-steroidal anti-inflammatory drugs (NSAIDs) for the prevention and treatment of postoperative ileus and gastric stasis.

A. Luckey et al. in Arch. Surg. Vol. 138, pages 206-214 (February 2003) present a review article that describes the state of the art in the treatment and prevention of postoperative ileus. A large number of alternative treatments are discussed. These include a variety of pharmacologic agents including NSAIDs.

M. Pairet et al. in J. Pharm. Pharmacol. Vol 41, pages 757-761 (1989) describe both oral and intraperitoneal administration of NSAIDS for the prevention of postoperative ileus. There is no disclosure of local administration to the site of the surgery.

US-A-20040109894 describes controlled release microspheres for selective release of therapeutic agents in different regions of the gastrointestinal tract following oral administration.

### Summary

The present invention relates to compositions and methods for preventing or reducing gastric stasis and postoperative ileus, which compositions and methods involve direct administration of therapeutic agents to the serosal surfaces of the affected organs. The invention is directed to compositions suitable for administration to the serosal surfaces of the gastrointestinal and other visceral organs to prevent or reduce postoperative ileus and gastric stasis. Such compositions comprise a carrier component and a bioactive component, which acts to prevent or reduce POI.

Specifically, the present invention provides a composition for use in a method of reduction or prevention of postoperative ileus and gastric stasis, which method comprises local application of said composition to the intestinal and other visceral surfaces in an area that has been manipulated by the surgeon, wherein said composition comprises: a carrier component in a form selected from the group consisting of an injectable gel, a sprayable gel, microparticles, beads, a mesh, a weave, a knit, and a nonwoven, provided that said carrier component does not comprise oxidized regenerated cellulose; and a bioactive component, wherein said bioactive component comprises a nonsteroidal anti-inflammatory drug selected from the group consisting of propionic acid derivatives, acetic acid derivatives, fenamic acid derivatives, biphenylcarboxylic acid derivatives, alkali metal salts thereof, and combinations thereof.

These and other aspects and advantages of the present invention will be more apparent from the following description and accompanying drawings.

### Brief Description of the Drawings

Fig. 1 is a perspective view of one embodiment of the present invention depicting a sheet containing a pharmaceutical agent effective in reducing POI;
Fig. 2 is a perspective view of another embodiment of the present invention depicting a sheet with a plurality of voids containing a pharmaceutical agent effective in reducing POI;
Fig. 3 is a side elevational view of a laparoscopy instrument useful in positioning a sheet of surgical material containing a pharmaceutical agent effective in reducing POI;
Fig. 4 is a perspective view of another embodiment of the present invention including an adhesive;
Fig. 5 is a flowchart illustrating a method of applying a pharmaceutical agent effective in reducing POI to the outside membrane of the intestine using an endoscope;
Fig. 6 is an elevation view of an applicator for applying a pharmaceutical agent effective in reducing POI;
Fig. 7 is a top plan view of a buttress containing a pharmaceutical agent effective in reducing POI;
Fig. 8 is a perspective view of the bioabsorbable circular staple buttress with buttresses containing a pharmaceutical agent effective in reducing POI;
Fig. 9 is a flowchart illustrating a method of applying a pharmaceutical agent at an operation site using an anastomotic device;
Fig. 10 is a side perspective view of a linear-type surgical stapler fitted with a pharmaceutical agent effective in reducing POI; and
Fig. 11 is a cross-sectional view of a surgical staple coated with a pharmaceutical agent effective in reducing POI.

### Detailed Description

To overcome the challenges of systemic administration of bioactive that reduces or prevents POI, the compositions of the present invention locally delivers a bioactive to the affected site, such as the irritated serosal lining. By incorporating the bioactive component into the carrier component the underlying pathophysiology of POI can be addressed without the side effects and toxicity issues of systemic delivery.

Specifically, the drug is delivered to the area that has been manipulated by the surgeon in doses that are lower than the systemic daily doses required to achieve analgesia. By lowering the systemic doses, the side effects are also reduced.

The present invention is directed to compositions and methods for preventing or reducing POI by direct administration of a composition comprising a carrier component and a bioactive component which acts to prevent or reduce POI to the serosal sufaces of the gastrointestinal and other visceral organs.

### Carrier Component

The carrier component of the compositions of the present invention is used to locally deliver the bioactive component to the site of the surgery. The carrier component can be in a number of physical forms including, injectable or sprayable gels, microparticles, beads and textile forms such as meshes, weaves, knits, and non-wovens. The bioactive component could be incorporated into the carrier component or coated onto the carrier component. Using gels, meshes, powders, and other means that can deliver such drugs locally will have the beneficial effect of reducing the incidence and severity of ileus, while not reaching plasma levels that will induce side effects such as sedation, or interfering with other systemic drugs that the patient is on.

In one embodiment, the carrier component is an injectable or sprayable gel. The injectable or sprayable gel is comprised of an aqueous solvent and a gelling material. Suitable aqueous solvents include, but are not limited to physiological buffer solution, saline and water such as, buffered saline, hypertonic saline, phosphate buffer solution, hypertonic buffer, Hank's balanced salts solution, Tris buffered saline, and Hepes buffered saline. In one embodiment, the aqueous solvent is phosphate buffer solution.

Suitable gelling materials include, proteins such as, collagen, elastin, thrombin, fibronectin, gelatin, fibrin, tropoelastin, polypeptides, laminin, proteoglycans, fibrin glue, fibrin clot, platelet rich plasma (PRP) clot, platelet poor plasma (PPP) clot, self-assembling peptide hydrogels, and atelocollagen; polysaccharides such as, starch, pectin, cellulose, alkyl cellulose, e.g. methylcellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, cross-linked alginate alginic acid, propylene glycol alginate, glycogen, dextran, dextran sulfate, curdlan, pectin, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, chitosan, starch, amylose, amylopectin, poly-N-glucosamine, polymannuronic acid, polyglucuronic acid polyguluronicacid, and derivatives; polynucleotides such as, ribonucleic acids, deoxyribonucleic acids, and others such as, poly(N-isopropylacrylamide), poly(oxyalkylene), copolymers of poly(ethylene oxide)-poly(propylene oxide), poly(vinyl alcohol), polyacrylate, monostearoyl glycerol co-succinate/polyethylene glycol (MGSA/PEG) copolymers and combinations thereof.

In one embodiment, the gelling material includes polysaccharides. In another embodiment, the gelling material includes sodium carboxymethylcellulose.

The injectable or sprayable gel may be prepared by dissolving an effective amount of gelling material in the aqueous solvent. An "effective amount" of gelling material is defined as the amount of gelling material sufficiently necessary to allow the injectable or sprayable gel or liquid to be either injected into or sprayed onto the affected area and stay in place upon application. The effective amount of gelling material will vary depending upon the material chosen. One of skill in the art may easily determine an effective amount of gelling material for the desired material. In one embodiment, where the gelling material is sodium carboxy methyl cellulose the gelling material is present in an amount of about 0.1 weight % to about 5 weight % in the aqueous solvent. In another embodiment, the gelling material is present in an amount of about 0.5 weight % to about 3 weight % in the aqueous solvent. The injectable or sprayable gel or liquid may be in a gel form prior to injection or may be in a liquid form and gel and stay in place upon administration.

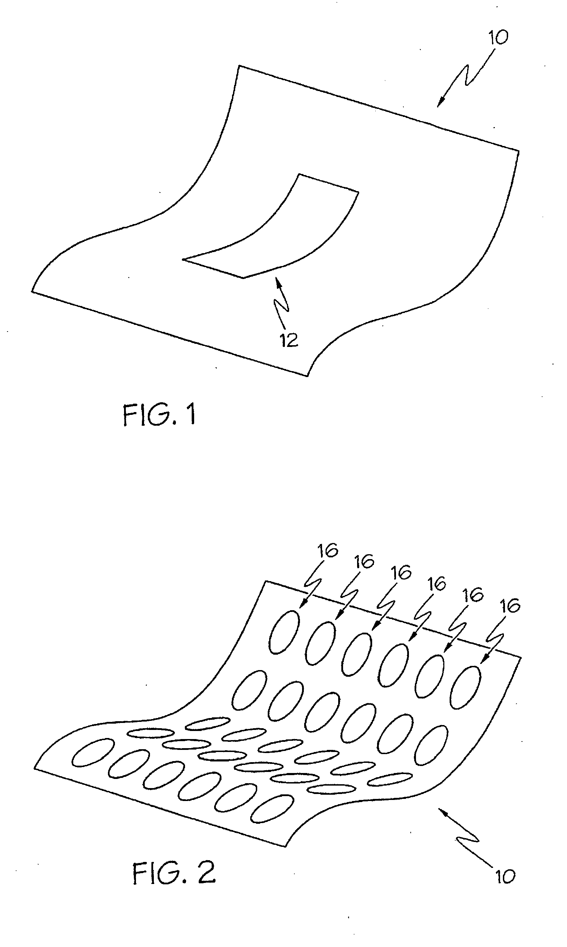

As illustrated in FIG. 1, one embodiment of the present invention is a sheet of surgical material 10 containing a pharmaceutical agent effective in reducing POI. The pharmaceutical agent effective in reducing POI is a NSAID. The NSAID to be used is to be applied in a pharmaceutically effective amount. The pharmaceutically effective amount will varying depending upon various factors including the type of surgery, the method and size of the incision, suturing, or stapling, the method used to apply the NSAID composition, and the form of the NSAID (hence forth "drug') composition. The drug composition may be applied in the form of a sheet, foam, powder, sponge, slurry, liquid, spray, mesh, netting, polymer coating, gel, or the like. The drug composition typically is applied to the interior or exterior of the intestine.

In one embodiment the sheet of surgical material **10** containing a pharmaceutical agent effective in reducing POI may contain a void **12** as shown in FIG. **1****.** The void **12** may be an area of lower concentration drug, or an opening cut into the sheet of surgical material **10.** The void **12** may be any geometric shape. Preferredly, the geometric shape of the void **12** is such that the sheet of surgical material **10** may be applied locally near the incision to administer an effective amount of drug at a desired location. The void **12** may be aligned with the patient's incision so the drug does not contact the incision, so as not to alter the body's normal healing process.

FIG. 2 shows a sheet of surgical material **10** containing a pharmaceutical agent effective in reducing POI with a plurality of voids **16.** The plurality of voids **16** may be formed by varying the concentration of drug across the sheet of surgical material **10** resulting in areas of little or no drug and/or ORC. The plurality of voids **16** may be formed by cutting openings into the sheet of surgical material. The plurality of voids **16** may be any geometric shape, and the geometric shapes may vary across the sheet of surgical material **10.** In one embodiment the plurality of voids **16** may be so numerous as to cause the sheet to function as a mesh.

The sheets of surgical material **10** containing the pharmaceutical agent effective in reducing POI shown in FIG. 1-2 may be attached to the desired location within the abdominal cavity or the intestine using, staples, clips, sutures, or the like, or the sheet material may be self-adhesive.

### Bioactive Component

The compositions and methods of the present invention involve a carrier component used to locally deliver a bioactive component to the site of the surgery.

The bioactive components are non-steroidal, anti-inflammatory drugs (NSAIDs). In one embodiment, the bioactive component functions as an inhibitor of the activity of cyclooxygenase (Cox1 and/or Cox2) enzymes referred to herein as NSAID Cox inhibitors. The NSAID Cox inhibitors can be those which are either non-selective or selective. Non-selective Cox inhibitors are those which inhibit the activity of both the Cox1 and Cox2 variants of the cyclooxygenase enzyme. Selective Cox inhibitors are those which selectively inhibit the activity of either the Cox1 or Cox2 enzyme forms preferentially.

Suitable NSAID Cox inhibitors include, but are not limited to propionic acid derivatives; acetic acid derivatives; fenamic acid derivatives; biphenylcarboxylic acid derivatives; and combinations thereof. These acids are sometimes administered in the form of their pharmaceutically acceptable acid or in the form of their alkali metal salts, e.g., sodium salts.

Propionic acid derivatives include, ibuprofen, naproxen, ketorolac, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen bucloxic acid and the like. Structurally related propionic acid derivatives having similar cyclooxygenase inhibiting properties are also intended to be encompassed by this group.

Acetic acid derivatives include, indomethacin, sulindac, tolmetin, zomepirac, diclofenac, bromofenac, fenclofenac, alclofenac, ibufenac, isoxepac, furofenac, tiopinac, zidometacin, acemetacin, fentiazac, clidanac oxpinac, and the like. Structurally related acetic acid derivatives having similar cyclooxygenase inhibiting properties are also intended to be encompassed by this group.

Fenamic acid derivatives include, mefenamic acid, meclofenamic acid, flufenamic acid, niflumic acid tolfenamic acid and the like. Structurally related fenamic acid derivatives having similar cyclooxygenase inhibiting properties are also intended to be encompassed by this group.

Biphenylcarboxylic acid derivatives include, diflunisal, flufenisal, and the like. Structurally related biphenylcarboxylic acid derivatives having similar cyclooxygenase inhibiting properties are also intended to be encompassed by this group.

In one embodiment, the NSAID Cox inhibitors include, ibuprofen, naproxen, flurbiprofen, fenoprofen, ketoprofen, fenbufen, zomepirac, diclofenac, ketorolac, bromofenac, indomethacin, mefenamic acid, meclofenamate acid, diflunisal, flufenisal, sodium salts thereof, and combinations thereof.

In another embodiment, the NSAID Cox inhibitors are acids having a secondary amine group. NSAID Cox inhibitors that are acids having a secondary amine group include, diclofenac; diclofenac, monosodium salt; mefenamic acid, monosodium salt; and bromofenac (bromide salt of diclofenac).

Cox inhibitors of all of the foregoing types, as well as other NSAIDs suitable as Cox inhibitors, are described in greater detail in United States Patent Nos. 6,689,382 and 6,231,888, United States Patent Publication No. 2003/0212050 and European Patent Application No. EP-A-485,111.

It is understood that the present invention contemplates the use of not only the NSAID Cox inhibitor compounds themselves, but also their pro-drugs which metabolize to the compounds and the analogs and biologically active salt forms thereof, as well as optical isomers which provide the same pharmaceutical results. Thus for purposes of this invention, the term "non-steroidal anti-inflammatory drug" is meant to include all derivatives or precursors such as salts, esters, pro-drugs, analogs, isomers, etc. which are NSAIDs themselves or which can yield materials which function as NSMDs.

### Composition Preparation Procedures and Preparation Adjuvants

The compositions herein can take a number of different forms depending upon the type and form of carrier component used to prepare the composition. Also, it may be appropriate to utilize various types of adjuvants in preparing such compositions in their several forms.

The carrier component is used to locally deliver the bioactive component to the site of the surgery. The bioactive component is incorporated into the carrier component. The bioactive component may be combined with the carrier component by manually mixing the components together or by conventional mechanical mixing such as, a motor driven rotating mixing paddle or blade. The mixing may be accomplished at ambient temperature. Using an injectable or sprayable gel or liquid that can deliver such drugs locally has the beneficial effect of reducing the incidence and severity of ileus, while not reaching plasma levels that will induce side effects such as sedation, or interfering with other systemic drugs that the patient is on.

The therapeutically effective concentration of a bioactive component will be one that is sufficient such that when delivered locally to the irritated or otherwise manipulated organ it will prevent or decrease the severity of post operative ileus. The maximum amount of the bioactive component will be limited by the toxicity of the bioactive component either at the local tissue or due to systemic levels.

The mass ratio of the bioactive component to the carrier component can typically range from about 1:50,000 to about 1:100. In one embodiment, this mass ratio of bioactive component to carrier component will range from about 1:30,000 to about 1:1,000.

The carrier component is used to locally deliver the bioactive component to the site of the surgery. The bioactive component is incorporated into the carrier component. Using an injectable or sprayable gel or liquid that can deliver such drugs locally has the beneficial effect of reducing the incidence and severity of ileus, while not reaching plasma levels that will induce side effects such as sedation, or interfering with other systemic drugs that the patient is on.

In one embodiment, where the carrier component is a textile, a solution of the bioactive component may be incorporated into the composition by (a) dip coating the textile in a solution or suspension of the bioactive component or (b) spray coating or adding dropwise a solution or suspension of the bioactive component onto the textile.

In addition to textile forms, the carrier component may be in the form of particles.

The compositions may also benefit from having a variety of optional The compositions may also benefit from having a variety of optional substances included such as stabilizers, wetting agents, or preservatives. Other drugs may also be added to the composition, so long as it is compatible with the bioactive component and the remaining ingredients. These drugs include antibiotics, antiviral, and anti-fungal agents. Since tissue sites are occasionally infected, an antibiotic or anti-microbial agent may also be combined with the composition.

The mechanism of release of the bioactive component from the carrier component should be in accordance with the development and maintenance of postoperative ileus. Hence, a release over 3-5 days will be preferable. Release over a shorter period of time is also acceptable, provided that the bioactive components prevent or reduce the development of ileus following the surgical intervention.

### Use of Composition to Prevent or Reduce Postoperative Ileus and Gastric Stasis

The methods of the present invention provide for administering directly to the serosal surfaces of the gastrointestinal or other visceral organs a therapeutically effective amount of a composition of the present invention as hereinbefore described. Such a composition utilizes a carrier component for local delivery of a bioactive component which acts to prevent or reduce POI. Compositions, as described herein, are useful in variety of abdominal, urogynecoligical, and cardio-thoracic surgeries.

In the case of a composition comprising an injectable or sprayable carrier component, such as liquids, gels, or microparticulates/bead and a bioactive component, the composition can be either injected into or sprayed onto the surgical site during surgery or post-surgical manipulation and prior to closure of the surgical site. The composition may be applied by injecting or spraying the composition onto the serosal surfaces of the gastrointestinal or other visceral organs.

The compositions of the present invention containing a pharmaceutical agent effective in reducing POI may be formed into a gel, slurry, bead, sponge, or the like. A gel, slurry, bead, or sponge containing a pharmaceutical agent effective in reducing POI may be applied to the interior or the exterior of the intestine in a manner consistent with those described for powders by using an instrument that can pump or push the composition to the desired location within the patient. United States patent 6,251,063 teaches a delivery method and gun or syringe that can be used to introduce the gel, slurry, bead, sponge, or the like. The needle tip of the gun or syringe may be fitted with a wider blunt tip as needed to apply the varying thickness and consistencies for the gel, slurry, bead, sponge, or the like. Additionally, using the method taught in United States patent 6,251,063 the compositions may be injected into the wall of the intestine to reduce POI.

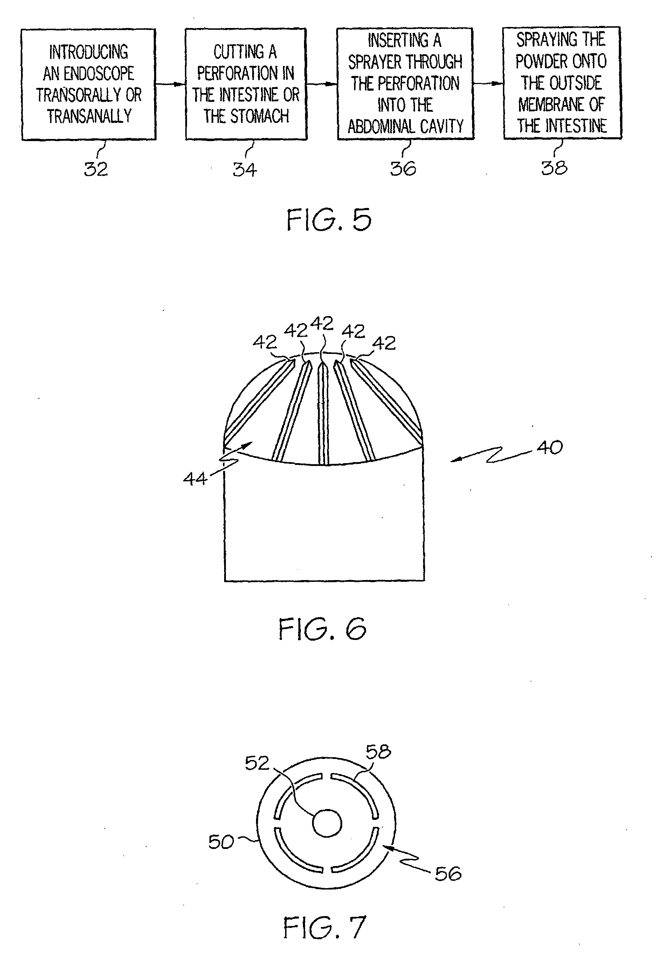

FIG. 6 shows a roll-on applicator **40** that may be filled with a gel or slurry composition containing a pharmaceutical agent effective in reducing POI. Using the roll-on applicator **40** the NSAID may be applied to the desired location of the intestine to reduce POI. The roll-on applicator **40** could be smooth, or in one embodiment be provided with multiple channels or groves, shown as v-shaped grooves **42** in the surface of the applicator tip **44**. The grooves or channels **42** need not be v-shaped, but may assume any desired shape so long as the function of channeling the gel or slurry composition containing a NSAID to the application area is achieved. For example, the grooves or channels **42** may be v-shaped, u-shaped, square, semi-circular, semi-oval, or any other geometric shape. The grooves or channels **42** may be formed such that they are exposed on the surface, or they may be formed to be located beneath the surface of the applicator tip **44.** Additionally, the applicator tip **44** need not be dome-shaped, as shown in FIG. 6, but may be any suitable applicator tip shape that applies the gel or slurry composition containing a NSAID to the area to be treated for POI or gastric stasis.

The roll-on applicator may be used in laparoscopic surgery, open surgery, or the like. In another embodiment a laparoscopic device with a rigid or flexible shaft of varying length may be fitted with the roll-on applicator. The laparoscopic device may be used to reach into the patient's body to apply the NSAID at the desired location.

The compositions of the present invention effective in reducing POI may be packaged as shown in United States patent 6,372,313. In one embodiment, the kit may contain the NSAID or mast cell degranulation inhibitor composition in a container, bottle, ampoule, pouch, sealed individual depressions, or the like of various sizes. The kit may also contain appliers of various sizes and shapes. The NSAID composition may be a solution capable of being placed onto an applier drop by drop. The NSAID may be a solution, slurry, or gel capable of having the applier dipped into the container and extracted containing a small amount of the NSAID ready for application to the patient's intestine or abdominal cavity. The NSAID may be a solid, powder, slurry, gel, solution, or the like stored in an ampoule that comes with an applier as shown in United States patent 6,340,097. The ampoule may have a shield that separates the NSAID composition from the air and must be broken by the applier to reach the composition.

The NSAID composition may need to be sterilized before use in treating POI. The NSAID or mast cell degranulation inhibitor composition may be packaged according to the methods in United States patent 6,412,639. The NSAID composition can be sterilized separately from the surgical tools and then remain sterile by separating the NSAID composition from the sterilization used for the surgical tools by way of a sterilization barrier built into the kit.

In the case of a composition comprising a textile carrier component and a bioactive component, the composition can be applied as a pledget material or draped over or around the affected surgical site.

In the case of meshes or textiles and powders, it is preferable that these adhere to the serosal lining and release the drug locally. The loading dose of the bioactive component can be in the range of 0.0016 to 0.16 g/cm² (0.01-10 mg/inch²) of area that comes into contact with the manipulated tissue. Following adherence it is preferred that the surface abutting other organs becomes lubricious so as to prevent adherence. It is preferable that foam carriers be supple and conform to the geometry of the organs they are attached to.

The composition may also be exposed to an exogenous source of aqueous fluid, preferably sterile phosphate buffered saline, immediately prior to application so as to aid in delivering the composition. Other suitable aqueous solutions that may be used just prior to implantation include sterile forms of Ringers solution, saline, and dextrose solution. In certain of the forms described above, the flowability of the wet composition allows it to conform to irregularities, crevices, cracks, or folds in the serosal tissue site.

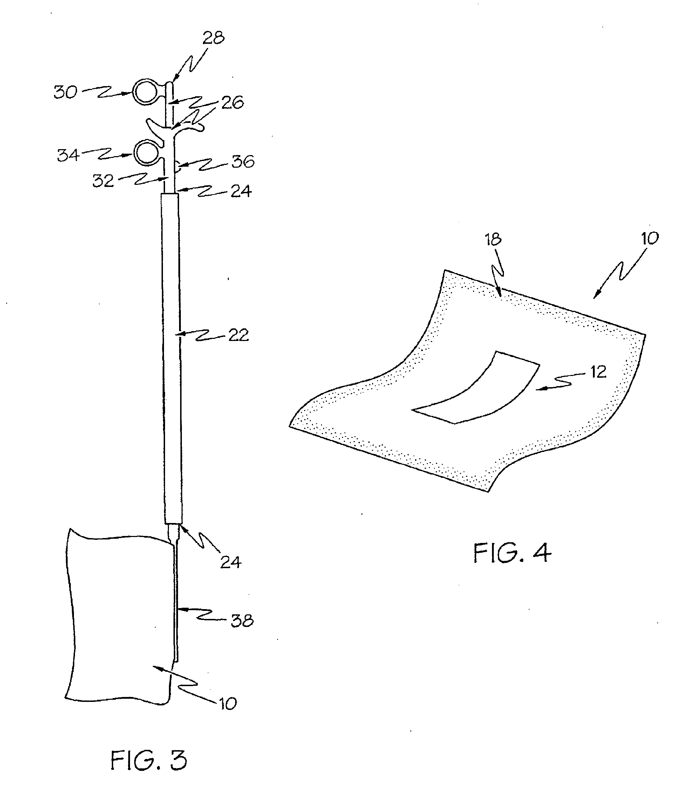

As illustrated in FIG. 3, a laparoscopy instrument **20** as described in United States Patent 5,503,623 may be used in the method for reducing post-operative ileus and/or gastric stasis to apply the sheet of surgical **material 10** containing the pharmaceutical agent effective in reducing POI to the intestine or abdominal cavity. The laparoscopy instrument **20** includes a tubular member **22** having a uniform cylindrical bore **24,** and an elongated inserter instrument **26** that can be placed through the abdominal wall. The elongated inserter instrument **26** is comprised of an external tubular member **32** and an internal tubular member **28.** Both the internal and external tubular members **28, 32** have a handle **30, 34** respectively for manipulating the member. The external tubular member **32** has a lever **36** that is used to articulate the grasping portion **38** into multiple angular positions.

To apply the effective amount of a pharmaceutical agent effective in reducing POI, a selected edge of the sheet of surgical material **10** containing the pharmaceutical agent effective in reducing POI is clamped securely within the grasping portion **38** and the surgeon then furls the sheet around the grasping portion **38**. The elongated inserter instrument **26** with the sheet of surgical material **10** containing the pharmaceutical agent effective in reducing POI furled around the grasping portion **38** may then by inserted through the tubular member **22** into the abdominal cavity. The sheet of surgical material 10 containing the pharmaceutical agent effective in reducing POI may then be unfurled inside the abdominal cavity and applied to the desired site for reducing POI.

The laparoscopy instrument **20** may also be used for the removal of a sheet of surgical material **10** containing a pharmaceutical agent effective in reducing POI. The above method of operating the laparoscopy instrument **20** is simply reversed such that the elongated inserter instrument is inserted into the abdominal cavity and while in the abdominal cavity a sheet of surgical material **10** containing a pharmaceutical agent effective in reducing POI is clamped into the grasping portion **38,** furled around the grasping portion **38,** and removed out through the tubular member **20.**

FIG. 4 shows another embodiment of a sheet of surgical material **10** containing a pharmaceutical agent effective in reducing POI that has an adhesive substance **18** applied over at least a portion of a side of the sheet, using methods described in United States Patent application US2005/0182443. The adhesive substance **18** may be applied on a single portion or a plurality of portions of the sheet of surgical material **10**, and may be applied in either a continuous or discontinuous manner. The adhesive substance **18** may be any suitable adhesive, but preferably, the adhesive substance **18** is a medical grade adhesive that is compatible with the pharmaceutical agent effective in reducing POI and any other compounds or ingredients in the composition. For example, the adhesive substance **18** may be an acrylic based pressure sensitive adhesives, rubber based pressure sensitive adhesives, silicone pressure adhesives, mixtures thereof, or the like as taught in United States Patent application US2005/0182443.

### Powder Mixtures.

In another embodiment the pharmaceutical agent effective in reducing POI may be applied to the abdominal cavity or the intestine as a powder to reduce post-operative ileus.

Such a powder may be applied to the mucosa or serosal layer of the intestine. The powder may be sprayed using a sprayer similar in form or effect to any of the various sprayers taught in United States Patent application US2005/0220721. For example, the sprayer may be a handgrip sprayer that includes a squeeze bulb positioned in the sprayer's grip that has an inlet valve and a conduit connected to a powder reservoir, a standard aerosol canister that includes a pressurized gas and an internal reservoir capable of holding a powder, a squeeze bulb sprayer with a powder reservoir positioned such that it may be inserted and removed without inverting the sprayer device.

The powder composition containing a pharmaceutical agent effective in reducing POI may be introduced transanally, transorally, transgastrically, or any combination thereof using such a sprayer. One embodiment of the method of applying the pharmaceutical agent effective in reducing POI as a powder may be by spraying the intestinal mucosa layer using any of the various sprayers fitted with a flexible tube of the necessary length to reach the desired location within the patient where the powder is to be applied.

One method of applying the powder using the sprayer is shown in FIG. 5. An endoscope is introduced transorally or transanally 32 into the intestine or stomach. A perforation is cut into the intestine or the stomach 34. Generally, when an endoscope is introduced transorally the perforation is cut into the stomach and when introduced transanally the perforation is cut into the intestine. The endoscope can be moved through the perforation and provide a channel for allowing other instruments to pass through the perforation as taught in United States patent 5,297,536. A sprayer, as described above, may be inserted through the perforation into the abdominal cavity 36. The sprayer may need to be fitted with a long flexible tubing to reach the operation site or the site to be treated to reduce POI. A powder containing a NSAID composition, as described above, may be sprayed into the abdominal cavity and onto the outside membrane of the intestines 38. In one embodiment, the sprayer's flexible tubing may be used through a working channel of the endoscope, a channel adjacent to the endoscope, or attached to a guide rail or tube attached to the endoscope.

Another embodiment of the method of reducing post-operative ileus and/or gastric stasis applies a pharmaceutical agent effective in reducing POI as a composition on the buttress material used in a surgical procedure or has the buttress material made of the composition. The design of the buttress material and the configuration of the NSAID composition on or within the buttress will vary with the surgical instrument that applies the buttress, the area to be treated, the patient being treated, and other medical variables.

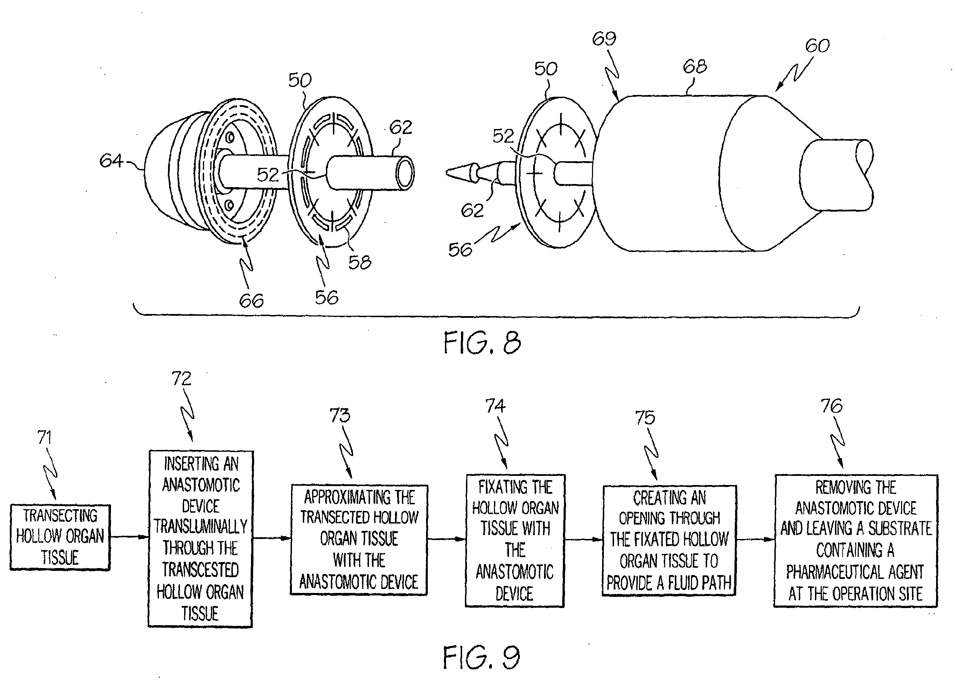

FIG. 8 is a perspective view of a typical circular surgical stapler **60** with two stapler buttresses **50** mounted on a central shaft 62 as described in United States patent application US 2005/0059996. The stapler has an anvil head **64** with a staple compression surface 66. As FIG. 8 shows, the anvil head **64** is removably attached to the stapler body **68** via the central shaft **62.** The stapler body also has a compression surface **69** through which staples are ejected. One or both buttresses **50** may contain an NSAID presenting surface **56**. When the buttresses **50** are placed on the central shaft **62,** the NSAID presenting surface **56** will be facing away from the staple compression surface **66** and away from the staple body compression surface **69** respectively if both buttresses **50** are used. If the buttresses **50** are made of the NSAID composition, then any orientation of the buttresses **50** on the central shaft **62** will provide for the application of an effective amount of the NSAID. When the circular surgical stapler **60** is fired the buttresses **50** will come together with the two pieces of intestine to be joined sandwiched between them (also known as completing an anastomosis between two hollow organs), which will put the NSAID into contact with the intestine.

One embodiment of buttress material **50** shown in FIG. 7 is for use with a circular surgical stapler **60** as shown in FIG. 8. The buttress material **50** has a central opening **52** so the buttress material **50** will fit into the circular surgical stapler **60.** The buttress **50** contains a NSAID composition. The NSAID composition **58** shown in FIG. 7 will allow the staples to be in the outer most ring of the buttress. In one embodiment the NSAID composition **58** is in the outer most ring and the staples are in the next closest interior ring. Another embodiment has the NSAID and the staples together in the same ring, even though some clinicians believe the NSAID may decrease the body's natural healing inflammation along the staple or incision line.

In one embodiment the buttress material **50** may include the NSAID in the overall composition of the buttress material **50**.. The NSAID may be any of the compounds described above. The concentration of the NSAID within the buttress material **50** may vary across the buttress and may be arranged in any configuration necessary to treat the effected area.

In another embodiment the NSAID may be applied to the buttress material **50** using any technique that will effectively hold the NSAID to the buttress material. The NSAID may be applied to one or both sides of the buttress material **50.** When the NSAID is applied to only one side of the buttress material **50**, that side is called the NSAID presenting surface **56**. The concentration of the NSAID on the buttress material **50** may vary across the buttress. The NSAID may cover all or part of the buttress material **50**, and may be any geometric shape or concentration necessary to treat the affected area of the patient for POI or gastric stasis and to conform to the surgical instrument applying the buttress.

FIG. 9 shows a flowchart of a method for transecting hollow organ tissue **71** to fixate tissue together using an anastomotic device **74** containing a substrate capable of capturing a pharmaceutical agent effective in reducing POI that will be left at the operation site **76**. An anastomotic device is inserted transluminally through the transected hollow organ tissue **72**, and approximated to the transected hollow organ tissues **73**. The anastomotic device contains at least one mechanical fastener or other fixation mechanism for fixating the tissue and a substrate capable of capturing a pharmaceutical agent. The anastomotic device is then used to fixate the tissue **74**. An opening is created through the fixated tissue to provide a fluid path 75. Then the anastomotic device is removed leaving the substrate containing the pharmaceutical agent effective in reducing POI behind at the operation site **76**.

The substrate may be a sheet, mesh, buttress, or any other substrate that is compatible with the anastomotic device. The pharmaceutical agent effective in reducing POI may be any of the NSAIDs listed above. More preferredly the NSAID is diclofenac or a pharmaceutically acceptable salt thereof.

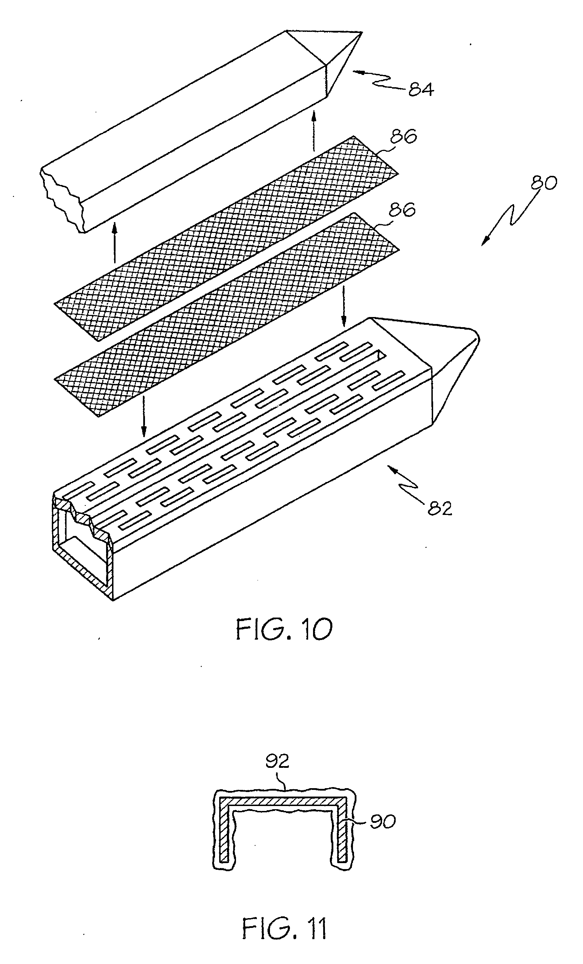

FIG. 10 is a side perspective view of a linear-type surgical stapler **80** with a substrate **86** containing a pharmaceutical agent effective in reducing POI. The NSAID may be applied to the substrate **86** on one side, the NSAID surface **87**, both sides, or made part of the substrate material **86.** The linear-type surgical stapler **80** can utilize one or a plurality of the substrate **86**. The linear-type surgical stapler **80** has a staple cartridge **82** with a channel 88 therein, and an anvil **84**.

The substrate **86** containing a NSAID is positioned against the anvil **84** and/or staple cartridge **82** respectively with the NSAID surface **87** facing each other. The substrate **86** may be a sheet, mesh, or the like as described in FIGS. 1-2, 4. The substrate **86** may be buttress material or any other substrate material shaped to fit the linear-type surgical stapler **80**. In one embodiment, the substrate **86** is caprolactone/glycolid, but may be any of the delivery agents listed above.

The sheet, mesh, buttress or substrate contain a NSAID as the pharmaceutical agent. The NSAID may be any of the compounds listed above, but more preferredly is diclofenac or a pharmaceutically acceptable salt thereof. The concentration of the NSAID may vary across the substrate **86** creating areas of low concentration and areas of high concentration. The NSAID may be concentrated in any geometric shape necessary to treat the effected area of the patient for POI or gastric stasis and to conform to the surgical instrument applying the substrate **86**. In one embodiment, the NSAID is concentrated in an area separate from the staples, but may be concentrated with the staples even though the NSAID may decrease the body's natural healing inflammation along the staple line.

FIG. 11 shows a surgical staple **90** that has a coating **92** containing an effective amount of a composition containing a NSAID or mast cell degranulation inhibitor to reduce POI. In one embodiment, the surgical staple **90** may be made of a composition containing a NSAID or mast cell degranulation inhibitor or have the composition containing the NSAID or mast cell degranulation inhibitor impregnated into it. The staple may be an absorbable staple, a metal staple, or any other staple material capable of containing, holding, absorbing, or being coated with a NSAID or mast cell degranulation inhibitor. Preferably the composition containing the NSAID or mast cell degranulation inhibitor also contains a polymer, such as polyacrylic acid, hydroxylpropyl methyl cellulose, polyvinyl pyrrolidone, polyethylene glycol, and polyethylene oxide. The NSAID composition may also contain a variety of other substances such as stabilizers, wetting agents, or preservatives. Other drugs may also be added to the composition, so long as they are compatible with the NSAID and any other compounds or ingredients. These drugs include but are not limited to antibiotics, antiviral, anti-fungal, and anti-microbial agents.

### Packaging Kits

The pharmaceutical agent effective in reducing POI may be packaged as shown in United States patent 6,372,313. In one embodiment, the kit may contain the NSAID composition in a container, bottle, ampoule, pouch, sealed individual depressions, or the like of various sizes. The kit may also contain appliers of various sizes and shapes. The NSAID composition may be a solution capable of being placed onto an applier drop by drop. The NSAID may be a solution, slurry, or gel capable of having the applier dipped into the container and extracted containing a small amount of the NSAID ready for application to the patient's intestine or abdominal cavity. The NSAID may be a solid, powder, gel, or the like stored in an ampoule that comes with an applier as shown in United States patent 6,340,097. The ampoule may have a shield that separates the NSAID composition from the air and must be broken by the applier to reach the composition.

The NSAID composition may need to be sterilized before use in treating POI. The NSAID composition may be packaged according to the methods in United States patent 6,412,639. The NSAID composition can be sterilized separately from the surgical tools and then remain sterile by separating the NSAID composition from the sterilization used for the surgical tools by way of a sterilization barrier built into the kit.

The following examples are illustrative of the principles and practice of the present invention, although not limited thereto.

### EXAMPLES

To illustrate the present invention, compositions containing various forms of carrier components and bioactive components are prepared. Combinations of carrier components and bioactive components, such as those used in the compositions and methods of this invention, are also evaluated via *in vivo* testing for their ability to alleviate postoperative ileus.

### Example 1

### Preparation of Diclofenac Sodium loaded gel

Sodium carboxymethylcellulose (Na-CMC, Type 7H3SFPH, Hercules, Willmington, DE) gels were loaded with Diclofenac sodium (DFNa, cat.# D6899-100G, Sigma-Aldrich, St. Louis, MO) using a physical mixing. Two drug loadings (high dosage and low dosage) were prepared. Ten grams of Na-CMC were slowly added into 290 grams of phosphate buffered saline (PBS) solution with magnetic stirring at 60 °C to prepare 3 wt% Na-CMC solution. The pre-mixed solution was autoclaved using AMSCO Steam Powered Sterilizer (Model #3021, SN 0100593-08) at 121 °C for 20 minutes to make a homogenous gel solution. The high dosage gel composition (1mg DFNa/3g Na-CMC), was prepared by adding 13 mg of DFNa to 39 grams of 3 wt% Na-CMC gel. The mixture was well mixed manually with a spatula for 5 minutes. 3 grams of DFNa high dose composition were loaded into 5cc disposable syringes. The low dosage gel composition (0.3mg DFNa/3g Na-CMC), was prepared by adding 3.9 mg of DFNa to 39 grams of 3wt% Na-CMC gel. The mixture was well mixed by hands for 5 minutes. 3 grams of DFNa low dose composition were loaded into 5cc disposable syringes. 3 grams of 3 wt% Na-CMC gel were loaded into 5cc disposable syringes for the gel only treatment.

### In vivo Testing of the effects of Diclofenac gel on POI

The charcoal transit model is an accetable and established model to study the effects of abdominal surgery on intestinal motility. We followed the procedure as described by De Winter et al. (1997) (Reference: Effect of adrenergic and nitrergic blockade on experimental ileus in rats. British Journal of Pharmacology (1997) 120, 464 - 468. Benedicte Y. De Winter, Guy E. Boeckxstaens, Joris G. De Man, Tom G. Moreels,Arnold G. Herman & Paul A. Pelckmans). The model simulates the abdominal procedure in human and tests for gastric emptying and intestinal motility by the transfer of a charcoal meal fed to the animals.

### Methods of Treatment

### Surgeries

Male Sprague Dawley rats, weighing 250-275g (8 in group) were anesthetized with a xylazine/ketamine cocktail, injected IM, the abdomen was shaved and disinfected with alcohol and Betadyne. A 2.5 cm incision was made from the xyphoid process caudally. The intestinal contents was exteriorized onto wet gauze. The cecum was manipulated gently for 1 minute. The abdominal contents were replaced, the treatment was applied, and the body wall was closed with 4-0 PDS for the fascia and muscle layers. The skin was closed with wound clips. In addition, two control groups were tested as well: incision only and naive. Treatment groups included two control groups, surgery (incision only) and naïve, and two treatment groups, a high and low dose of Diclofenac, as well as 3 wt % Na-CMC gel alone. The high and low dose of Diclofenac, as well as the 3 wt% Na-CMC gel were prepared as described above. 8 rats were included in each group with a total of 40 rats.

### Charcoal transit

The rats were treated with an oral solution of charcoal (10%) in acacia gum (2%). Four hours following oral administration rats were euthanized with Euthasole. The intestines were exteriorized and the distance traveled by the charcoal meal was measured as well as the full length of the intestines (from the pylorus through the anus). Results are expressed as percent of distance traveled relative to the full length of the intestines.

### Study Groups

| **Drug** | **Gel composition** | |
|---|---|---|
| | **Dose (mg/ml)** | **Total dose (mg)** |
| Gel only | 0.00 | 0 |
| Diclofenac-Na low | 0.10 | 0.3 |
| Diclofenac-Na high | 0.33 | 1 |
| | | |

### Results

| Study Group | Dose (mg) | % GI Transfer | SE |
|---|---|---|---|
| Naive (no surgery) | NA | 92.63 | 0.76 |
| Control (surgery - no treatment) | NA | 37.79 | 1.01 |
| Gel only | NA | 38.76 | 1.81 |
| Diclofenac-Na low | 1.0 | 95.05 | 1.23 |
| Diclofenac-Na high | 0.3 | 73.58 | 0.90 |

It was observed that intestinal manipulation decreases gastric emptying and intestinal motility in the rat in the same manner that has been described in humans undergoing intestinal surgery. Diclofenac sodium, when delivered via a gel, to the manipulated intestines, prevents the decrease in gastric emtying and GI motility in a dose related manner. A complete prevention was observed when the high dose was used and a significant prevention of POI was observed when the low dose was used. The gel alone had no significant effect on the POI.

### Example 2

### Naproxen loaded gel

Na-CMC gels loaded with Naproxen (lot#:076K15861 Sigma-Aldrich, St. Louis, MO) were prepared using physical mixing. Two loadings (high dosage and low dosage) were prepared. 10 grams of Na-CMC were slowly added into 290 gram of PBS solution with magnetic stirring at 60 °C to prepare 3 wt% Na-CMC solution. The pre-mixed solution was autoclaved at 121 °C for 20 minutes to make a homogenous gel solution.

The high dosage gel composition (2mg Naproxen/3g Na-CMC) was prepared by adding24 mg ofNaproxen to 36 g of 3 wt% Na-CMC gel. The mixture was manually mixed with a spatula for 5 minutes. 3 g of gel were weighed into 5 ml syringes with a 3 way stopcock. Syringes were autoclaved for 20 minutes at 121°C using an AMSCO Steam Powered Sterilizer(Model #3021, SN 0100593-08).

The low dosage gel composition (0.3mg Naproxen/3g Na-CMC) was prepared by adding 3.6 mg of Naproxen to 36 g of 3wt% Na-CMC gel. The mixture was manually mixed using a spatula for 5 minutes. 3 g of gel were weighed into 5ml syringes with a 3 way stopcock. Syringes were autoclaved for 20 minutes at 121°C using an AMSCO Steam Powered Sterilizer( Model #3021, SN 0100593-08).

### Ketorolac loaded gel

Na-CMC gels loaded with Ketorolac (lot#:083K0734, Sigma-Aldrich, St. Louis, MO) were prepared using physical mixing. Two loadings (high dosage and low dosage) were prepared. 10 grams of Na-CMC were slowly added into 290 gram of PBS solution with magnetic stirring at 60 °C to prepare 3 wt% Na-CMC solution. The pre-mixed solution was heated up to 121 °C for 20 minutes to make a homogenous gel solution. The high dosage gel composition (2mg Ketorolac/3g Na-CMC) was prepared by adding 24 mg of Ketorolac to 36 g of 3 wt% Na-CMC gel. The mixture was manually mixed with a spatula for 5 minutes. 3 g of gel were weighed into 5 ml syringes with a 3-way stopcock. Syringes were autoclaved for 20 minutes at 121°C using an AMSCO Steam Powered Sterilizer, Model #3021, SN 0100593-08. The low dosage gel composition (0.3mg Ketorolac /3g Na-CMC) was prepared by adding 3.6 mg of Ketorolac to 36 g of 3 wt% Na-CMC gel. The mixture was manually mixed using a spatula for 5 minutes. 3 g of gel were weighed into 5 ml syringes with a 3 way stopcock. Syringes were autoclaved for 20 minutes at 121°C using AMSCO Steam Powered Sterilizer, (Model #3021, SN 0100593-08).

### In vivo Testing of the effects of Ketorolac gel and Naproxen gel on POI

The charcoal transit model is an acceptable and established model to study the effects of abdominal surgery on intestinal motility. We followed the procedure as described by De Winter et al. (1997) (Reference: Effect of adrenergic and nitrergic blockade on experimental ileus in rats. British Journal of Pharmacology (1997) 120, 464 - 468. Benedicte Y. De Winter, Guy E. Boeckxstaens, Joris G. De Man, Tom G. Moreels,Arnold G. Herman & Paul A. Pelckmans). The model simulates the abdominal procedure in human and tests for gastric emptying and intestinal motility by the transfer of a charcoal meal fed to the animals.

### Methods of Treatment

### Surgeries

Male Sprague Dawley rats, weighing 250-275g (8 in group) were anesthetized with a xylazine/ketamine cocktail, injected IM, the abdomen was shaved and disinfected with alcohol and Betadyne. A 2.5 cm incision was made from the xyphoid process caudally. The intestinal contents was exteriorized onto wet gauze. The cecum was manipulated gently for 1 minute. The abdominal contents were replaced, the treatment was applied, and the body wall was closed with 4-0 PDS for the fascia and muscle layers. The skin was closed with wound clips. In addition, two control groups were tested as well: incision only and naïve. Treatment groups included two control groups, surgery (incision only) and naïve, and 4 treatment groups, a high and low dose of Diclofenac, a high and a low dose of Naproxen, as well as 3 wt % Na-CMC gel alone. The high and low dose of Diclofenac, as well as the 3 wt% Na-CMC gel were prepared as described above. 8 rats were included in each group with a total of 56 rats.

### Charcoal transit

The rats were treated with an oral solution of charcoal (10%) in acacia gum (2%). Four hours following oral administration rats were euthanized with Euthasole. The intestines were exteriorized and the distance traveled by the charcoal meal was measured as well as the full length of the intestines (from -34-the pylorus through the anus). Results are expressed as percent of distance traveled relative to the full length of the intestines.

### Study Groups

### Results

| Study Group | Dose (mg) | % GI Transfer | SE |
|---|---|---|---|
| Naïve (no surgery) | NA | 83.38 | 2.51 |
| Control (surgery - no treatment) | NA | 53.92 | 2.88 |
| Ketorolac high | 2.0 | 69.84 | 3.65 |
| Ketorolac low | 0.3 | 70.99 | 4.62 |
| Naproxen high | 2.0 | 65.34 | 3.03 |
| Naproxen low | 0.3 | 56.9 | 2.5 |
| Gel only | NA | 52.04 | 4.29 |

It was observed that intestinal manipulation decreases gastric emptying and intestinal motility in the rat in the same manner that has been described in humans undergoing intestinal surgery. A dose response was not observed using the current Ketorolac low and high dose formulations, but a significant alleviation of the POI was observed in both doses. Similarly, when the naproxen was applied to the manipulated intestines, a dose related prevention of the POI was observed. Unlike the diclofenac, where the high dose completely alleviated the POI, the high dose of naproxen was not completely curative, yet, both doses improved on the gastric emptying and intestinal transfer compared with the non-treated animals and the animals treated with the gel alone. The gel alone had no significant effect on the POI.

Although this invention has been shown and described with respect to detailed embodiments thereof, it will be understood by those skilled in the art that various changes in form and detail thereof may be made without departing from the scope of the claimed invention as defined in the accompanying claims.

## Claims

1. A composition for use in a method of reduction or prevention of postoperative ileus and gastric stasis, which method comprises local application of said composition to the intestinal and other visceral surfaces in an area that has been manipulated by the surgeon, wherein said composition comprises:
a carrier component in a form selected from the group consisting of an injectable gel, a sprayable gel, microparticles, beads, a mesh, a weave, a knit, and a nonwoven, provided that said carrier component does not comprise oxidized regenerated cellulose; and
a bioactive component, wherein said bioactive component comprises a nonsteroidal anti-inflammatory drug selected from the group consisting of propionic acid derivatives, acetic acid derivatives, fenamic acid derivatives, biphenylcarboxylic acid derivatives, alkali metal salts thereof, and combinations thereof.

2. The composition for use in a method of Claim 1, wherein said nonsteroidal anti-inflammatory drug is selected from the group consisting of ibuprofen, naproxen, ketorolac, benoxaprofen, flurbiprofen, fenoprofen, fenbufen, ketoprofen, indoprofen, pirprofen, carprofen, oxaprozin, pranoprofen, miroprofen, tioxaprofen, suprofen, alminoprofen, tiaprofenic acid, fluprofen bucloxic acid, indomethacin, sulindac, tolmetin, zomepirac, diclofenac, bromofenac, fenclofenac, alclofenac, ibufenac, isoxepac, furofenac, tiopinac, zidometacin, acemetacin, fentiazac, clidanac oxpinac, mefenamic acid, meclofenamic acid, flufenamic acid, niflumic acid tolfenamic acid, diflunisal, flufenisal, alkali metal salts thereof, and combinations thereof.

3. The composition for use in a method of Claim 2 wherein said nonsteroidal anti-inflammatory drug is selected from the group consisting of ibuprofen, naproxen, flurbiprofen, fenoprofen, ketoprofen, fenbufen, zomepirac, diclofenac, ketorolac, bromofenac, indomethacin, mefenamic acid, meclofenamic acid, diflunisal, flufenisal, sodium salts thereof, and combinations thereof.

4. The composition for use in a method of Claim 3 wherein said nonsteroidal anti-inflammatory drug is selected from the group consisting of diclofenac; diclofenac, monosodium salt; mefenamic acid, monosodium salt ; bromofenac and combinations thereof.

5. The composition for use in a method of any preceding claim, wherein said injectable gel or sprayable gel comprises an aqueous solvent and a gelling material.

6. The composition for use in a method of Claim 5 wherein said aqueous solvent is selected from the group consisting of physiological buffer solution, saline and water.

7. The composition for use in a method of Claim 5 wherein said aqueous solvent is selected from the group consisting of buffered saline, hypertonic saline, phosphate buffer solution, hypertonic buffer, Hank's balanced salts solution, Tris buffered saline, Hepes buffered saline and combinations thereof.

8. The composition for use in a method of Claim 5 wherein said gelling material is selected from the group consisting of collagen, elastin, thrombin, fibronectin, gelatin, fibrin, tropoelastin, polypeptides, laminin, proteoglycans, fibrin glue, fibrin clot, platelet rich plasma (PRP) clot, platelet poor plasma (PPP) clot, self-assembling peptide hydrogels, atelocollagen, starch, pectin, cellulose, alkyl cellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, cross-linked alginate, alginic acid, propylene glycol alginate, glycogen, dextran, dextran sulfate, curdlan, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, amylose, amylopectin, poly-N-glucosamine, polymannuronic acid, polyglucuronic acid, polyguluronicacid, ribonucleic acids, deoxyribonucleic acids, poly(N- isopropylacrylamide), poly(oxyalkylene), copolymers of poly(ethylene oxide)-poly(propylene oxide), poly(vinyl alcohol), polyacrylate, monostearoyl glycerol co-Succinate/polyethylene glycol (MGSA/PEG) copolymers and combinations thereof.

9. The composition for use in a method of Claim 8 wherein said gelling material is selected from the group consisting of starch, pectin, cellulose, alkyl cellulose, alkylhydroxyalkyl cellulose, hydroxyalkyl cellulose, cellulose sulfate, salts of carboxymethyl cellulose, carboxymethyl cellulose, carboxyethyl cellulose, chitin, carboxymethyl chitin, hyaluronic acid, salts of hyaluronic acid, alginate, cross-linked alginate, alginic acid, propylene glycol alginate, glycogen, dextran, dextran sulfate, curdlan, pullulan, xanthan, chondroitin, chondroitin sulfates, carboxymethyl dextran, carboxymethyl chitosan, chitosan, heparin, heparin sulfate, heparan, heparan sulfate, dermatan sulfate, keratan sulfate, carrageenans, amylose, amylopectin, poly-N-glucosamine, polymannuronic acid, polyglucuronic acid, polyguluronicacid and combinations thereof.

10. The composition for use in a method of Claim 9 wherein said gelling material is selected from the group consisting of salts of carboxymethyl cellulose, carboxymethyl cellulose, carboxyethyl cellulose, hyaluronic acid, salts of hyaluronic acid, alginate, cross-linked alginate and combinations thereof.

11. The composition for use in a method of Claim 10 wherein said gelling material comprises salts of carboxymethyl cellulose wherein said salts of carboxymethyl cellulose is sodium carboxymethyl cellulose.

12. The composition for use in a method of Claim 1 wherein said carrier component comprises an injectable gel comprising phosphate buffered saline and sodium carboxymethyl cellulose.

13. The composition for use in a method of Claim 1 wherein said carrier component comprises an injectable gel comprising phosphate buffered saline and sodium carboxymethyl cellulose; and wherein said bioactive component is diclofenac.

## Patentansprüche

1. Zusammensetzung zur Verwendung bei einem Verfahren zur Verminderung oder Prävention von postoperativem Ileus und Magenstase, wobei das Verfahren die lokale Anwendung der Zusammensetzung an den von dem Chirurgen behandelten Darm- und anderen viszeralen Oberflächen umfasst, wobei die Zusammensetzung Folgendes umfasst:
eine Trägerkomponente in einer Form ausgewählt aus der Gruppe bestehend aus einem injizierbaren Gel, einem sprühbaren Gel, Mikropartikeln, Kügelchen, einem Netz, einer Webware, einer Maschenware und einem Vliesstoff, mit der Maßgabe, dass die Trägerkomponente keine oxidierte regenerierte Cellulose umfasst, und
eine biologisch aktive Komponente, wobei die biologisch aktive Komponente ein aus der aus Propionsäurederivaten, Essigsäurederivaten, Fenaminsäurederivaten, Biphenylcarbonsäurederivaten, Alkalisalzen davon und Kombinationen davon bestehenden Gruppe ausgewähltes nichtsteroidales entzündungshemmendes Arzneimittel (Nonsteroidal Anti-Inflammatory Drug) umfasst.

2. Zusammensetzung zur Verwendung bei einem Verfahren nach Anspruch 1, wobei das nichtsteroidale entzündungshemmende Arzneimittel ausgewählt ist aus der Gruppe bestehend aus Ibuprofen, Naproxen, Ketorolac, Benoxaprofen, Flurbiprofen, Fenoprofen, Fenbufen, Ketoprofen, Indoprofen, Pirprofen, Carprofen, Oxaprozin, Pranoprofen, Miroprofen, Tioxaprofen, Suprofen, Alminoprofen, Tiaprofensäure, Fluprofen, Bucloxinsäure, Indomethacin, Sulindac, Tolmetin, Zomepirac, Diclofenac, Bromofenac, Fenclofenac, Alclofenac, Ibufenac, Isoxepac, Furofenac, Tiopinac, Zidometacin, Acemetacin, Fentiazac, Clidanac, Oxpinac, Mefenaminsäure, Meclofenaminsäure, Flufenaminsäure, Nifluminsäure, Tolfenaminsäure, Diflunisal, Flufenisal, Alkalisalzen davon und Kombinationen davon.

3. Zusammensetzung zur Verwendung bei einem Verfahren nach Anspruch 2, wobei das nichtsteroidale entzündungshemmende Arzneimittel ausgewählt ist aus der Gruppe bestehend aus Ibuprofen, Naproxen, Flurbiprofen, Fenoprofen, Ketoprofen, Fenbufen, Zomepirac, Diclofenac, Ketorolac, Bromofenac, Indomethacin, Mefenaminsäure, Meclofenamatsäure, Diflunisal, Flufenisal, Natriumsalzen davon und Kombinationen davon.

4. Zusammensetzung zur Verwendung bei einem Verfahren nach Anspruch 3, wobei das nichtsteroidale entzündungshemmende Arzneimittel ausgewählt ist aus der Gruppe bestehend aus Diclofenac, Diclofenac-Mononatriumsalz, Mefenaminsäure-Mononatriumsalz, Bromofenac und Kombinationen davon.

5. Zusammensetzung zur Verwendung bei einem Verfahren nach einem der vorhergehenden Ansprüche, wobei das injizierbare Gel oder sprühbare Gel ein wässriges Lösungsmittel und ein Geliermaterial umfasst.

6. Zusammensetzung zur Verwendung bei einem Verfahren nach Anspruch 5, wobei das wässrige Lösungsmittel ausgewählt ist aus der Gruppe bestehend aus physiologischer Pufferlösung, Kochsalzlösung und Wasser.

7. Zusammensetzung zur Verwendung bei einem Verfahren nach Anspruch 5, wobei das wässrige Lösungsmittel ausgewählt ist aus der Gruppe bestehend aus gepufferter Kochsalzlösung, hypertoner Kochsalzlösung, Phosphatpufferlösung, hypertonem Puffer, Hank's Balanced Salts Solution, Tris-gepufferter Kochsalzlösung, Hepes-gepufferter Kochsalzlösung und Kombinationen davon.

8. Zusammensetzung zur Verwendung bei einem Verfahren nach Anspruch 5, wobei das Geliermaterial ausgewählt ist aus der Gruppe bestehend aus Kollagen, Elastin, Thrombin, Fibronektin, Gelatine, Fibrin, Tropoelastin, Polypeptiden, Laminin, Proteoglycanen, Fibrinleim, Fibrinkoagulat, Koagulat von thrombozytenreichem Plasma (Platelet Rich Plasma, PRP), Koagulat von thrombozytenarmem Plasma (Platelet Poor Plasma, PPP), selbstassemblierenden Peptidhydrogelen, Atelokollagen, Stärke, Pektin, Cellulose, Alkylcellulose, Alkylhydroxyalkylcellulose, Hydroxalkylcellulose, Cellulosesulfat, Salzen von Carboxymethylcellulose, Carboxymethylcellulose, Carboxyethylcellulose, Chitin, Carboxymethylchitin, Hyaluronsäure, Salzen von Hyaluronsäure, Alginat, quervernetztem Alginat, Algensäure, Propylenglykolalginat, Glycogen, Dextran, Dextransulfat, Curdlan, Pektin, Pullulan, Xanthan, Chondroitin, Chondroitinsulfaten, Carboxymethyldextran, Carboxymethylchitosan, Chitosan, Heparin, Heparinsulfat, Heparan, Heparansulfat, Dermatansulfat, Keratansulfat, Carrageenen, Chitosan, Stärke, Amylose, Amylopectin, Poly-N-glucosamin, Polymannuronsäure, Polyglucuronsäure, Polyguluronsäure, Ribonukleinsäuren, Desoxyribonukleinsäuren, Poly(N-isopropylacrylamid), Poly(oxyalkylen), Copolymeren von Poly(ethylenoxid)-poly(propylenoxid), Poly(vinylalkohol), Polyacrylat, Monostearoylglycerol-cosuccinat/polyethylenglykol-(MGSA/PEG)-Copolymeren und Kombinationen davon.

9. Zusammensetzung zur Verwendung bei einem Verfahren nach Anspruch 8, wobei das Geliermaterial ausgewählt ist aus der Gruppe bestehend aus Stärke, Pektin, Cellulose, Alkylcellulose, Alkylhydroxyalkylcellulose, Hydroxyalkylcellulose, Cellulosesulfat, Salzen von Carboxymethylcellulose, Carboxymethylcellulose, Carboxyethylcellulose, Chitin, Carboxymethylchitin, Hyaluronsäure, Salzen von Hyaluronsäure, Alginat, quervernetztem Alginat, Algensäure, Propylenglykolalginat, Glykogen, Dextran, Dextransulfat, Curdlan, Pektin, Pullulan, Xanthan, Chondroitin, Chodroitinsulfaten, Carboxymethyldextran, Carboxymethylchitosan, Chitosan, Heparin, Heparinsulfat, Heparan, Heparansulfat, Dermatansulfat, Keratansulfat, Carrageenen, Chitosan, Stärke, Amylose, Amylopektin, Poly-N-glucosamin, Polymannuronsäure, Polyglucuronsäure, Polyguluronsäure und Kombinationen davon.

10. Zusammensetzung zur Verwendung bei einem Verfahren nach Anspruch 9, wobei das Geliermaterial ausgewählt ist aus der Gruppe bestehend aus Salzen von Carboxymethylcellulose, Carboxymethylcellulose, Carboxyethylcellulose, Hyaluronsäure, Salzen von Hyaluronsäure, Alginat, quervernetztem Alginat und Kombinationen davon.

11. Zusammensetzung zur Verwendung nach Anspruch 10, wobei das Geliermaterial Salze von Carboxymethylcellulose umfasst, wobei es sich bei den Salzen von Carboxymethylcellulose um Natriumcarboxymethylcellulose handelt.

12. Zusammensetzung zur Verwendung bei einem Verfahren nach Anspruch 1, wobei die Trägerkomponente ein injizierbares Gel umfasst, welches phosphatgepufferte Kochsalzlösung und Natriumcarboxymethylcellulose umfasst.

13. Zusammensetzung zur Verwendung bei einem Verfahren nach Anspruch 1, wobei die Trägerkomponente ein injizierbares Gel umfasst, welches phosphatgepufferte Kochsalzlösung und Natriumcarboxymethylcellulose umfasst, und wobei es sich bei der biologisch aktiven Komponente um Diclofenac handelt.

## Revendications

1. Composition pour une utilisation dans une méthode de réduction ou de prévention d'une stase iléale et gastrique postopératoire, laquelle méthode comprend l'application locale de ladite composition à la surface intestinale et autres surfaces viscérales dans une zone ayant été manipulée par le chirurgien, **caractérisée en ce que** ladite composition comprend :
un composant véhicule sous une forme choisie dans le groupe constitué par un gel injectable, un gel pulvérisable, des microparticules, des billes, un tulle, un tissu tissé, un tissu tricoté et un tissu non tissé, à condition que ledit composant véhicule ne comprenne pas de cellulose régénérée oxydée ; et
un composant bioactif, **caractérisé en ce que** ledit composant bioactif comprend un anti-inflammatoire non stéroïdien choisi dans le groupe constitué par les dérivés d'acide propionique, les dérivés d'acide acétique, les dérivés d'acide fénamique, les dérivés d'acide biphénylcarboxylique, les sels de métaux alcalins de ceux-ci, et des combinaisons de ceux-ci.

2. Composition pour une utilisation dans une méthode selon la revendication 1, dans laquelle ledit anti-inflammatoire non stéroïdien est choisi dans le groupe constitué par l'ibuprofène, le naproxène, le kétorolac, le bénoxaprofène, le flurbiprofène, le fénoprofène, le fenbufène, le kétoprofène, l'indoprofène, le pirprofène, le carprofène, l'oxaprozine, le pranoprofène, le miroprofène, le tioxaprofène, le suprofène, l'alminoprofène, l'acide tiaprofénique, le fluprofène, l'acide bucloxique, l'indométhacine, le sulindac, la tolmétine, le zomépirac, le diclofénac, le bromofénac, le fenclofénac, l'alclofénac, l'ibufénac, l'isoxépac, le furofénac, le tiopinac, la zidométacine, l'acémétacine, le fentiazac, le clidanac, l'oxpinac, l'acide méfénamique, l'acide méclofénamique, l'acide flufénamique, l'acide niflumique, l'acide tolfénamique, le diflunisal, le flufénisal, les sels de métaux alcalins de ceux-ci, et des combinaisons de ceux-ci.

3. Composition pour une utilisation dans une méthode selon la revendication 2, dans laquelle ledit anti-inflammatoire non stéroïdien est choisi dans le groupe constitué par l'ibuprofène, le naproxène, le flurbiprofène, le fénoprofène, le kétoprofène, le fenbufène, le zomépirac, le diclofénac, le kétorolac, le bromofénac, l'indométhacine, l'acide méfénamique, l'acide méclofénamate, le diflunisal, le flufénisal, les sels de sodium de ceux-ci, et des combinaisons de ceux-ci.

4. Composition pour une utilisation dans une méthode selon la revendication 3, dans laquelle ledit anti-inflammatoire non stéroïdien est choisi dans le groupe constitué par le diclofénac ; le diclofénac, sel monosodique ; l'acide méclofénamique, sel monosodique ; le bromofénac ; et des combinaisons de ceux-ci.

5. Composition pour une utilisation dans une méthode selon l'une quelconque des revendications précédentes, dans laquelle ledit gel injectable ou gel pulvérisable comprend un solvant aqueux et un matériau gélifiant.

6. Composition pour une utilisation dans une méthode selon la revendication 5, dans laquelle ledit solvant aqueux est choisi dans le groupe constitué par une solution de tampon physiologique, une solution saline et de l'eau.

7. Composition pour une utilisation dans une méthode selon la revendication 5, dans laquelle ledit solvant aqueux est choisi dans le groupe constitué par une solution saline tamponnée, une solution saline hypertonique, une solution tamponnée au phosphate, un tampon hypertonique, une solution saline équilibrée de Hank, une solution saline tamponnée au Tris, une solution saline tamponnée au HEPES, et des combinaisons de ceux-ci.

8. Composition pour une utilisation dans une méthode selon la revendication 5, dans laquelle ledit matériau gélifiant est choisi dans le groupe constitué par le collagène, l'élastine, le thrombine, le fibronectine, la gélatine, la fibrine, la tropoélastine, les polypeptides, la laminine, les protéoglycanes, la colle de fibrine, le caillot de fibrine, le caillot de plasma riche en plaquettes (PRP), le caillot de plasma pauvre en plaquettes (PPP), les hydrogels de peptides auto-assemblés, l'atélocollagène, l'amidon, la pectine, la cellulose, l'alkylcellulose, l'alkylhydroxy alkyl-cellulose, l'hydroxyalkylcellulose, le sulfate de cellulose, les sels de carboxyméthylcellulose, la carboxyméthylcellulose, la carboxyéthylcellulose, la chitine, la carboxyméthyl chitine, l'acide hyaluronique, les sels de l'acide hyaluronique, l'alginate, l'alginate réticulé, l'acide alginique, l'alginate de propylène glycol, le glycogène, le dextran, le sulfate de dextran, le curdlan, le pullulane, le xanthane, la chondroïtine, les sulfates de chondroïtine, le carboxyméthyl dextran, le carboxyméthyl chitosane, le chitosane, l'héparine, le sulfate d'héparine, l'héparane, le sulfate d'héparane, le sulfate de dermatane, le sulfate de kératane, les carraghénanes, l'amylose, l'amylopectine, la poly-N-glucosamine, l'acide polymannuronique, l'acide polyglucuronique, l'acide polyguluronique, les acides ribonucléiques, les acides désoxyribonucléiques, le poly(N-isopropylacrylamide), le poly(oxyalkylène), les copolymères de poly(oxyde d'éthylène)-poly(oxyde de propylène), le poly(alcool vinylique), le polyacrylate, les copolymères de monostéaroylglycérol-co-succinate/polyéthylène glycol (MGSA/PEG), et des combinaisons de ceux-ci.

9. Composition pour une utilisation dans une méthode selon la revendication 8, dans laquelle ledit matériau gélifiant est choisi dans le groupe constitué par l'amidon, la pectine, la cellulose, l'alkylcellulose, l'alkylhydroxy alkylcellulose, l'hydroxyalkylcellulose, le sulfate de cellulose, les sels de carboxyméthylcellulose, la carboxyméthylcellulose, la carboxyéthylcellulose, la chitine, la carboxyméthyl chitine, l'acide hyaluronique, les sels de l'acide hyaluronique, l'alginate, l'alginate réticulé, l'acide alginique, l'alginate de propylène glycol, le glycogène, le dextran, le sulfate de dextran, le curdlan, le pullulane, le xanthane, la chondroïtine, les sulfates de chondroïtine, le carboxyméthyl dextran, le carboxyméthyl chitosane, le chitosane, l'héparine, le sulfate d'héparine, l'héparane, le sulfate d'héparane, le sulfate de dermatane, le sulfate de kératane, les carraghénanes, l'amylose, l'amylopectine, la poly-N-glucosamine, l'acide polymannuronique, l'acide polyglucuronique, l'acide polyguluronique, et des combinaisons de ceux-ci.

10. Composition pour une utilisation dans une méthode selon la revendication 9, dans laquelle ledit matériau gélifiant est choisi dans le groupe constitué par les sels de carboxyméthylcellulose, la carboxyméthylcellulose, la carboxyéthylcellulose, l'acide hyaluronique, les sels de l'acide hyaluronique, l'alginate, l'alginate réticulé, et des combinaisons de ceux-ci.

11. Composition pour une utilisation dans une méthode selon la revendication 10, dans laquelle ledit matériau gélifiant comprend des sels de carboxyméthylcellulose, dans laquelle lesdits sels de carboxyméthylcellulose sont la carboxyméthylcellulose de sodium.

12. Composition pour une utilisation dans une méthode selon la revendication 1, dans laquelle ledit composant véhicule comprend un gel injectable comprenant une solution saline tamponnée au phosphate et de la carboxyméthylcellulose de sodium.

13. Composition pour une utilisation dans une méthode selon la revendication 1, dans laquelle ledit composant véhicule comprend un gel injectable comprenant une solution saline tamponnée au phosphate et de la carboxyméthylcellulose de sodium ; et dans laquelle ledit composant bioactif est le diclofénac.
